(19) Europäisches Patentamt
European Patent Office
Office européen des brevets

(11) **EP 4 089 114 A1**

(12) **EUROPEAN PATENT APPLICATION**
published in accordance with Art. 153(4) EPC

(43) Date of publication:
**16.11.2022 Bulletin 2022/46**

(21) Application number: **21738450.2**

(22) Date of filing: **06.01.2021**

(51) International Patent Classification (IPC):
***C07K 16/28*** (2006.01)   ***A61K 39/395*** (2006.01)
***A61P 35/00*** (2006.01)   ***A61K 39/00*** (2006.01)

(52) Cooperative Patent Classification (CPC):
**A61K 39/00; A61K 39/395; A61P 35/00;
C07K 16/28**

(86) International application number:
**PCT/CN2021/070493**

(87) International publication number:
**WO 2021/139687 (15.07.2021 Gazette 2021/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.01.2020   CN 202010023504**

(71) Applicant: **Innovent Biologics (Suzhou) Co., Ltd.
Suzhou, Jiangsu 215123 (CN)**

(72) Inventors:
• **CHEN, Bingliang
Suzhou, Jiangsu 215123 (CN)**
• **WU, Min
Suzhou, Jiangsu 215123 (CN)**
• **WU, Weiwei
Suzhou, Jiangsu 215123 (CN)**

(74) Representative: **Nederlandsch Octrooibureau
P.O. Box 29720
2502 LS The Hague (NL)**

Remarks:
The complete document including Reference
Table(s) and the Sequence Listing(s) can be
downloaded from the EPO website

(54) **APPLICATION OF COMBINATION OF ANTI-CD47 ANTIBODY AND ANTI-CD20 ANTIBODY IN PREPARATION OF DRUGS FOR PREVENTING OR TREATING TUMORS**

(57) The present invention discloses use of a combination of an anti-CD47 antibody or an antigen-binding fragment thereof and an anti-CD20 antibody in preparing a medicament for preventing and/or treating a tumor and/or cancer, wherein the anti-CD47 antibody is a fully human anti-CD47 monoclonal antibody. In addition, the present invention also discloses an anti-tumor pharmaceutical combination and use thereof. The anti-CD47 antibody or the antigen-binding fragment thereof provided herein in combination with the anti-CD20 antibody has excellent anti-tumor efficacy.

EP 4 089 114 A1

**Description**

**TECHNICAL FIELD**

The present invention relates to the field of biotechnology, particularly to an anti-tumor pharmaceutical combination and use thereof, and more particularly to use of a combination of an anti-CD47 antibody or an antigen-binding fragment thereof and an anti-CD20 antibody in preparing a medicament for preventing and/or treating a tumor and/or cancer.

**BACKGROUND**

Rituximab is a monoclonal antibody targeting human CD20 molecule which can eliminate CD20-positive tumor cells by effects such as ADCC and CDC, and is currently used as a first-line treatment for non-Hodgkin's lymphoma. Although rituximab is superior as a treatment for non-Hodgkin's lymphoma, data have shown that resistance to rituximab may occur in patients due to reasons such as FcR polymorphism and loss of CD20 targets in patients, and that the tumor may become refractory and relapse.

Cluster of differentiation 47 (CD47), also known as an integrin-associated protein (IAP), is a member of the immunoglobulin superfamily. Different studies show that almost all tumor cells and tissues can highly express CD47. Multiple anti-CD47 antibodies have been reported to be developed for the treatment of various tumors and/or cancers.

Although Forty Seven, Inc. has anti-CD47 antibody and rituximab combination schemes and related products, it remains a challenge in the medical field to find a combination scheme and related product with feasibility and superior effect (reduced dose, improved incidence and/or severity of treatment emerged adverse events (AE), synergistic effect, etc.) to known combination schemes in view of factors such as the complexity of the tumorigenesis mechanism and the unpredictability of interaction between different anti-CD47 antibodies and rituximab. As such, there is still a necessity to develop an improved anti-tumor pharmaceutical combination with superior anti-tumor efficacy.

**BRIEF SUMMARY**

The present invention is intended to provide use of a combination of an anti-CD47 antibody and an anti-CD20 antibody in preparing a medicament for preventing and/or treating a tumor and/or cancer superior to the prior art.

In one aspect, the present invention provides use of a combination of an anti-CD47 antibody or an antigen-binding fragment thereof and an anti-CD20 antibody in preparing a medicament for preventing and/or treating a tumor and/or cancer, wherein the anti-CD47 antibody is a fully human anti-CD47 monoclonal antibody. The fully human anti-CD47 monoclonal antibody comprises a heavy chain variable region VH and a light chain variable region VL, wherein the VH comprises complementarity determining regions HCDR1, HCDR2 and HCDR3, the HCDR1 having an amino acid sequence set forth in SEQ ID NO: 3 or SEQ ID NO: 11, the HCDR2 having an amino acid sequence set forth in SEQ ID NO: 4, the HCDR3 having an amino acid sequence set forth in SEQ ID NO: 5 or SEQ ID NO: 12; the VL comprises complementarity determining regions LCDR1, LCDR2 and LCDR3, the LCDR1 having an amino acid sequence set forth in SEQ ID NO: 6, the LCDR2 having an amino acid sequence set forth in SEQ ID NO: 7, the LCDR3 having an the amino acid sequence set forth in SEQ ID NO: 8.

In some embodiments, in the above use, the heavy chain variable region VH of the fully human anti-CD47 monoclonal antibody comprises an amino acid sequence set forth in SEQ ID NO: 9 or a sequence having at least 90%, 95%, 98% or 99% identity thereto, and the light chain variable region comprises a sequence set forth in SEQ ID NO: 10 or a sequence having at least 90%, 95%, 98% or 99% identity thereto.

Preferably, the heavy chain variable region VH has an amino acid sequence set forth in SEQ ID NO: 9, and the light chain variable region VL has an amino acid sequence set forth in SEQ ID NO: 10.

In some embodiments, in the above use, the fully human anti-CD47 monoclonal antibody comprises a heavy chain comprising an amino acid sequence set forth in SEQ ID NO: 1 or an amino acid sequence having at least 90%, 95%, 98% or 99% identity thereto, and a light chain comprising an amino acid sequence set forth in SEQ ID NO: 2 or an amino acid sequence having at least 90%, 95%, 98% or 99% identity thereto.

Preferably, the heavy chain has an amino acid sequence set forth in SEQ ID NO: 1, and the light chain has an amino acid sequence set forth in SEQ ID NO: 2.

In some embodiments, the fully human anti-CD47 monoclonal antibody is the anti-CD47 monoclonal antibody disclosed in Patent Publication No. WO2019042285A1, which is incorporated herein by reference in its entirety for the purpose of the present application, wherein the anti-CD47 antibody is preferably the anti-CD47 antibody ADI-26630 disclosed in WO2019042285A1. The sequence of the anti-CD47 antibody in use is renumbered herein.

In some embodiments, in the above use, the anti-CD20 antibody is an anti-CD20 monoclonal antibody, such as obinutuzumab, ibritumomab tiuxetan, ofatumumab, tositumomab, ocrelizumab or rituximab.

In some embodiments, in the above use, the anti-CD20 monoclonal antibody is rituximab.

In another aspect, the present invention further provides a single pharmaceutical dosage unit comprising effective amounts of the anti-CD47 antibody or the antigen-binding fragment thereof and the anti-CD20 antibody according to any one of the above.

In another aspect, the present invention further provides a pharmaceutical composition comprising an effective amount of the combination of the anti-CD47 antibody or the antigen-binding fragment thereof and the anti-CD20 antibody according to any one of the above, and a pharmaceutically acceptable carrier.

In another aspect, the present invention further provides a kit of parts comprising the above pharmaceutical composition.

Preferably, the kit is in the form of a single pharmaceutical dosage unit.

In some embodiments, in the same package, the above kit comprises:

- a first container containing a pharmaceutical composition for parenteral administration comprising the anti-CD47 antibody according to any one of the above; and
- a second container containing a pharmaceutical composition for parenteral administration comprising the anti-CD20 antibody according to any one of the above.

In another aspect, the present invention further provides use of the pharmaceutical combination, the single pharmaceutical dosage unit or the kit of parts according to any one of the above in preparing a medicament for preventing and/or treating a tumor and/or cancer, preferably a hematological tumor, more preferably lymphoma, and even more preferably CD20-positive non-Hodgkin's lymphoma.

In another aspect, the present invention further provides use of the composition according to any one of the above in preparing a medicament for preventing and/or treating a tumor and/or cancer, preferably a hematological tumor, more preferably lymphoma, and even more preferably CD20-positive non-Hodgkin's lymphoma. In another aspect, the present invention further provides use of an anti-CD47 antibody in preparing a product for enhancing an efficacy of an anti-CD20 antibody in preventing and/or treating a tumor and/or cancer, preferably a hematological tumor, more preferably lymphoma, and even more preferably CD20-positive non-Hodgkin's lymphoma.

In another aspect, the present invention further provides a method for preventing and/or treating a tumor and/or cancer, comprising: administering to an individual an effective amount of the combination of the anti-CD47 antibody or the antigen-binding fragment thereof and the anti-CD20 antibody according to any one of the above, the single pharmaceutical dosage unit according to any one of the above, the pharmaceutical composition according to any one of the above or the kit of parts according to any one of the above.

The anti-CD47 antibodies described herein are novel fully human anti-CD47 monoclonal antibodies which, whether administered alone or in combination with anti-CD20 monoclonal antibody rituximab, show better anti-tumor efficacy than Hu5F9 of Forty Seven, Inc. Particularly, the anti-tumor efficacy of the combination of the fully human anti-CD47 monoclonal antibody and rituximab is significantly better than that of the combination of Hu5F9 and rituximab.

The present invention, by combining the anti-CD47 antibody described herein, facilitates the increase of macrophage-mediated phagocytosis and can improve the efficacy of rituximab. Compared with combinations of anti-CD47 antibody and rituximab in the prior art, the present invention shows superior anti-tumor efficacy and therefore has good prospects in effectively solving the resistance to rituximab in patients due to reasons such as FcR polymorphism and loss of CD20 targets.

**SUMMARY**

Definitions

The term "and/or" should be understood to refer to any one of the options or any two or more of the options.

The term "comprise" or "comprising" is intended to include the described elements, integers or steps, but not to exclude any other elements, integers or steps. As used herein, the term "comprise" or "comprising", unless indicated otherwise, also encompasses the situation where the entirety consists of the described elements, integers or steps. For example, when referring to an antibody variable region "comprising" a particular sequence, it is also intended to encompass an antibody variable region consisting of the particular sequence.

As used herein, the term "antibody" refers to a polypeptide comprising at least an immunoglobulin light chain variable region or heavy chain variable region that specifically recognizes and binds to an antigen. The term "antibody" encompasses a variety of antibody structures, including but not limited to, monoclonal antibodies, polyclonal antibodies, single-chain or multi-chain antibodies, monospecific or multispecific antibodies (e.g., bispecific antibodies), fully human or chimeric or humanized antibodies, and full-length antibodies and antibody fragments, so long as they exhibit the desired antigen-binding activity.

The term "antigen-binding fragment" (used interchangeably herein with "antibody fragment" and "antigen-binding portion") of the antibody refers to an incomplete antibody molecule that comprises a portion of an intact antibody for binding to an antigen to which the intact antibody binds. It will be understood by those skilled in the art that the antigen-binding portion of an antibody generally comprises amino acid residues from a "complementarity determining region" or a "CDR". The antigen-binding fragment may be prepared by recombinant DNA techniques, or by enzymatic or chemical cleavage of the complete antibody. The antigen-binding fragment includes, but is not limited to, an Fab, an scFab, an Fab', an F(ab')2, an Fab'-SH, an Fv, a single-chain Fv, a diabody, a triabody, a tetrabody, a minibody, and a single-domain antibody (sdAb). For more detailed descriptions of the antibody fragment, see: Fundamental Immunology, W.E. Paul eds., Raven Press, N.Y. (1993); Shao Rongguang et al. (eds.), Antibody Drug Research and Application, People's Medical Publishing House (2013); Hollinger et al., PNAS USA 90:6444-6448 (1993); Hudson et al., Nat. Med. 9:129-134 (2003).

The term "prevention" (or "prevent" or "preventing") includes the inhibition or delay of the onset or onset frequency of a disease or disorder or symptoms thereof, which generally refers to the administration of a drug prior to the onset of disorders or symptoms, particularly in an individual at risk.

The term "treatment" (or "treat" or "treating") used herein refers to the slowing, arresting or reversing of the progression of the cancer in a subject as evidenced by the reduction or elimination of clinical or diagnostic symptoms of the disease. Treatment may include, for example, reduced severity of symptoms, number of symptoms, or frequency of recurrence, e.g., inhibited tumor growth, arrested tumor growth, or regression of existing tumors.

The term "single pharmaceutical dosage unit" used herein represents a single pharmaceutical dosage form including injections, tablets, and lyophilized powders, administered to a patient at the time of a scheduled administration.

The term "pharmaceutical combination" refers to a non-fixed combination product or a fixed combination product, e.g., a kit. The term "non-fixed combination" means that active ingredients (e.g., (i) an anti-CD47 antibody or an antigen-binding fragment thereof, and (ii) an anti-CD20 antibody) are administered, either simultaneously, without specific time limitation or at identical or different time intervals, or sequentially, to a patient as separate entities, wherein such administration provides prophylactically or therapeutically effective levels of the two active agents in the patient. In some embodiments, the anti-CD47 antibody molecule and the anti-CD20 antibody molecule used in a pharmaceutical combination are administered at levels that do not exceed their levels when used alone. The term "fixed combination" means that two active agents are administered to a patient simultaneously in the form of a single entity. The dose and/or time intervals of the two active agents are preferably selected such that the combined use of the components results in a therapeutic effect on the disease or disorder which is greater than that achieved by the use of either component alone. The ingredients may each take a separate formulation form and such separate formulation forms may be the same or different.

The term "administration" (or "administer" or "administering") refers to physically introducing the active ingredients of the pharmaceutical combination of the present invention into an individual using any one of various methods and delivery systems known to those skilled in the art. Routes of administration for the active ingredients in the pharmaceutical combination of the present invention include oral, intravenous (e.g., infusion (also known as drip) or injection), intramuscular, subcutaneous, intraperitoneal, spinal, topical or other parenteral routes of administration. The term "parenteral administration" used herein refers to modes of administration other than gastrointestinal and topical administration, typically by intravenous injection and infusion, and includes, but is not limited to, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, and *in vivo* electroporation. Accordingly, the active ingredients in the pharmaceutical combination of the present invention may be formulated into capsules, tablets, injections (including infusion or injection solutions), syrups, sprays, lozenges, liposomes, suppositories or the like.

The term "dose" is an amount of a drug that induces a therapeutic effect. Unless otherwise stated, the dose is related to the amount of a drug in free form. If the drug takes the form of a pharmaceutically acceptable salt, the amount of the drug is increased proportionally relative to the amount of the drug in free form. For example, the dose will be stated on the product package or in the product information sheet.

The pharmaceutical combination of the present invention may be administered to an individual who has been treated with one or more prior therapies but subsequently undergone relapse or metastasis.

As used herein, the term "monoclonal antibody" refers to a preparation having an antibody molecule having a single amino acid composition rather than a method for production the same. Monoclonal antibodies or antigen-binding fragments thereof may be produced, for example, by hybridoma technology, recombinant technology, phage display technology, synthetic technology such as CDR grafting, or a combination of such or other technologies known in the art.

As used herein, the terms "binding" and "specific binding" refer to the binding of an antibody or an antigen-binding portion to an epitope in an *in vitro* assay, preferably in biological optical interferometry (ForteBio) adopting a purified wild-type antigen. In certain embodiments, when the antibodies or the antigen-binding portions preferably recognize target antigens thereof in a complex mixture of proteins and/or macromolecules, the antibodies or the antigen-binding portions are referred to as specific binding antigens.

The term "variable region" or "variable domain" refers to a domain of a heavy chain or light chain of an antibody involved in the binding of the antibody to an antigen. Variable domains of heavy and light chains of natural antibodies typically have similar structures, wherein each domain comprises four conservative framework regions (FRs) and three complementarity determining regions. (See, e.g., Kindt et al., Kuby Immunology, 6th Ed., W.H. Freeman and Co., p 91 (2007)). A single VH or VL domain may be sufficient to provide antigen-binding specificity. In addition, a VH or VL domain from an antibody binding to a particular antigen can be used to isolate antibodies that bind to the antigen, so as to screen libraries of complementary VL or VH domains. See, e.g., Portolano et al., J. Immunol., 150:880-887 (1993); Clarkson et al., Nature, 352:624-628 (1991).

Variable regions generally exhibit the same general structure of relatively conservative framework regions (FRs) connected by three highly variable regions, and the highly variable regions are also referred to as "complementarity determining regions" or "CDRs". CDRs from each pair of chains are generally aligned by the framework regions, such that the CDRs can bind to specific epitopes. A light chain variable region and a heavy chain variable region generally comprise domains FR1, CDR1, FR2, CDR2, FR3, CDR3 and FR4 from N terminus to C terminus.

A "complementarity determining region" or "CDR region" or "CDR" or "highly variable region" (used interchangeably herein with hypervariable region "HVR"), is a region in an antibody variable domain that is highly variable in sequence and forms a structurally defined loop ("hypervariable loop") and/or comprises antigen-contacting residues ("antigen contact site"). CDRs are primarily responsible for binding to antigen epitopes. The CDRs of heavy and light chains are generally referred to as CDR1, CDR2 and CDR3, which are numbered sequentially from N terminus. The CDRs located in a heavy chain variable domain of an antibody are referred to as HCDR1, HCDR2 and HCDR3, whereas the CDRs located in a light chain variable domain of an antibody are referred to as LCDR1, LCDR2 and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature, 342:877-883; Al-Lazikani et al., Standard conformations for the canonical structures of immunoglobulins, Journal of Molecular Biology, 273:927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International Im-MunoGeneTics database (IMGT) (imgt.cines.fr/ on the World Wide Web), and North CDR definition based on the affinity propagation clustering using a large number of crystal structures.

For example, Kabat- and Chothia-numbered CDR regions have different definition ranges.

Table 1

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

The exact boundary of CDRs of the antibody of the present invention can be determined according to any scheme in the art or a combination thereof, and according to human evaluation.

CDRs can also be determined based on positions having the same Kabat number as the reference CDR sequences.

Unless otherwise stated, the term "CDR" or "CDR sequence" used herein encompasses CDR sequences determined by any one of the schemes above.

Unless otherwise stated, in the present invention, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

In one embodiment, the boundaries of the CDRs of the antibody of the present invention are determined by the Kabat scheme, e.g., as shown in Table 2 below. In one embodiment, the boundaries of the CDRs of the antibody of the present invention are determined in consideration of factors such as the Kabat scheme, AbM scheme, Chothia scheme and experience, e.g., as shown in Table 2 below: positions RASQGISRWLA (LCDR1), positions AASSLQS (LCDR2) and positions QQTVSFPIT (LCDR3) in VL, and positions GSISSYYWS (HCDR1), positions YIYYSGSTNYNPSLKS (HCDR2) and positions ARGKTGSAA (HCDR3) in VH.

However, it should be noted that boundaries of CDRs of variable regions of an antibody based on different assignment systems may differ. That is, CDR sequences of variable regions of an antibody defined by different assignment systems differ. Accordingly, when it comes to defining an antibody with specific CDR sequences defined in the present invention, the scope of antibody also encompasses such antibodies whose variable region sequences comprise the specific CDR sequences, but having claimed CDR boundaries different from the specific CDR boundaries defined by the present invention due to a different scheme (e.g., different assignment system scheme or their combinations) applied. Antibodies with different specificities (i.e., different binding sites for different antigens) have different CDRs. However, although CDRs differ from antibody to antibody, only a limited number of amino acid positions within the CDRs are directly involved in antigen binding. The smallest overlapping region can be determined using at least two of the Kabat, Chothia, AbM, Contact and North methods, thereby providing a "minimal binding unit" for antigen binding. The minimal binding unit may be a sub-portion of the CDR. As will be clear to those skilled in the art, residues of the rest portions in the CDR sequences can be determined via antibody structure and protein folding. Therefore, any variants of the CDRs given herein are also considered in the present invention. For example, in a variant of one CDR, the amino acid residue of the minimal binding unit may remain unchanged, while the remaining CDR residues defined by the Kabat or Chothia may be conservatively substituted.

The calculation of sequence identity between sequences is performed as follows. To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the aligned length of the reference sequence is at least 30%, preferably at least 40%, more preferably at least 50% or 60%, and even more preferably at least 70%, 80%, 90% or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48:444-453; available at http://www.gcg.com) which has been integrated into the GAP program of the GCG software package, using the Blossom 62 matrix or PAM250 matrix and a gap weight of 16, 14, 12, 10, 8, 6 or 4 and a length weight of 1, 2, 3, 4, 5 or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70 or 80 and a length weight of 1, 2, 3, 4, 5 or 6. A particularly preferred parameter set (and one that should be used unless otherwise stated) is a Blossom 62 scoring matrix with a gap penalty of 12, a gap extension penalty of 4, and a frameshift gap penalty of 5.

The percent identity between two amino acid sequences or nucleotide sequences can also be determined with PAM120 weighted remainder table, a gap length penalty of 12 and a gap penalty of 4, using the E. Meyers and W. Miller algorithm ((1989) CABIOS, 4:11-17) which has been incorporated into the ALIGN program (version 2.0).

The effective amount can be easily determined by an attending physician as a person skilled in the art by considering a variety of factors as follows: species such as mammals; its size, age, and general health condition; specific disease involved; extent or severity of the disease; response in an individual patient; specific antibody administered; route of administration; bioavailability characteristics of the administered preparation; selected dosage regimen; and use of any concomitant therapy.

As described above, in some cases, the interaction between an antibody and a target antigen thereof will interfere with the function of the target. The amount required for administration further depends on the binding affinity of an antibody to a specific antigen thereof, as well as the clearance rate of the antibody given in a subject receiving the treatment. "Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human.

The term "anti-CD47 antibody", "anti-CD47", "CD47 antibody" or "antibody binding to CD47" refers to an antibody capable of binding to a CD47 protein or a fragment thereof with sufficient affinity so as to serve as a diagnostic agent and/or a therapeutic agent in targeting CD47.

Heavy chain sequence of the anti-CD47 antibody of the present invention:

QVQLQESGPGLVKPSETLSLTCTVSGGSISSYYWSWIRQPPGKGLEWIGYI
YYSGSTNYNPSLKSRVTISVDTSKNQFSLKLSSVTAADTAVYYCARGKTG
SAAWGQGTLVTVSSASTKGPSVFPLAPCSRSTSESTAALGCLVKDYFPEPV
TVSWNSGALTSGVHTFPAVLQSSGLYSLSSVVTVPSSSLGTKTYTCNVDH
KPSNTKVDKRVESKYGPPCPPCPAPEAAGGPSVFLFPPKPKDTLMISRTPE
VTCVVVDVSQEDPEVQFNWYVDGVEVHNAKTKPREEQFNSTYRVVSVL
TVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFL
YSRLTVDKSRWQEGNVFSCSVMHEALHNHYTQKSLSLSLG (SEQ ID NO: 1);

Light chain sequence of the anti-CD47 antibody of the present invention:

DIQMTQSPSSVSASVGDRVTITCRASQGISRWLAWYQQKPGKAPKLLIYA
ASSLQSGVPSRFSGSGSGTDFTLTISSLQPEDFATYYCQQTVSFPITFGGGT
KVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLS
SPVTKSFNRGEC (SEQ ID NO: 2).

The sequences of the heavy chain CDRs and light chain CDRs of the anti-CD47 antibody of the present invention are shown in Table 2. The sequences of the heavy chain variable regions and light chain variable regions of the anti-CD47 antibody of the present invention are shown in Table 3.

Table 2. Sequences of heavy chain CDRs and light chain CDRs of the anti-CD47 antibody of the present invention

| Combinations | HCDR1 | HCDR2 | HCDR3 | LCDR1 | LCDR2 | LCDR3 |
|---|---|---|---|---|---|---|
| (1) (Determined according to the Kabat scheme) | SYYWS (SEQ ID NO:11) | YIYYSGSTNYN PSLKS (SEQ ID NO:4) | GKTGSAA (SEQ ID NO:12) | RASQGISRWLA (SEQ ID NO:6) | AASSLQS (SEQ ID NO:7) | QQTVSFPIT (SEQ ID NO:8) |
| (2) (Determined empirically) | GSISSYYWS (SEQ ID NO:3) | YIYYSGSTNYN PSLKS (SEQ ID NO:4) | ARGKTGSAA (SEQ ID NO:5) | RASQGISRWLA (SEQ ID NO:6) | AASSLQS (SEQ ID NO:7) | QQTVSFPIT (SEQ ID NO:8) |

Table 3. Sequences of heavy chain variable regions and light chain variable regions of the anti-CD47 antibody of the present invention

| Name | Heavy chain variable region (VH) | Light chain variable region (VL) |
|---|---|---|
| Anti-CD47 antibody | QVQLQESGPGLVKPSETLSLTCTVSGGSISSYYWS WIRQPPGKGLEWIGYIYYSGSTNYNPSLKSRVTIS VDTSKNQFSLKLSSVTAADTAVYYCARGKTGSAA WGQGTLVTVSS (SEQ ID NO: 9) | DIQMTQSPSSVSASVGDRVTITCRASQGISRWLAW YQQKPGKAPKLLIYAASSLQSGVPSRFSGSGSGTD FTLTISSLQPEDFATYYCQQTVSFPITFGGGTKVEI K (SEQ ID NO: 10) |

## BRIEF DESCRIPTION OF THE DRAWINGS

FIG. 1 shows the body weight change rates of tumor-bearing mice in various groups.
FIG. 2 shows the changes in tumor volume of tumor-bearing mice in various groups.
FIG. 3 shows the changes in tumor volume of tumor-bearing mice in low dose groups.
FIG. 4 shows the changes in tumor volume of tumor-bearing mice in high dose groups.

## DETAILED DESCRIPTION

Unless otherwise specified, the experimental procedures used in the following examples are all conventional procedures.

Unless otherwise specified, materials, reagents and the like used in the following examples are commercially available.

The present invention is further illustrated below in conjunction with specific examples. It should be understood that the following examples are merely intended to illustrate the present invention rather than limit the scope of the present invention. The Raji lymphoma cells were purchased from ATCC under Catalog No. CCL-86TM, Batch No. 58770576.

The Matrigel gel was purchased from CORNING, U.S. under Catalog No. 356231. The NOD-SCID mice (female, aged 35-41 days) were purchased from Beijing Vital River Laboratory Animal Technology Co., Ltd. under Certificate No. 1100111911006865.

h-IgG was purchased from Equitech-Bio under Catalog No. SLH56, Batch No. 160308-02.

## Example 1. Production and Isolation of Anti-CD47 Antibody, Hu5F9 and Rituximab

1. Anti-CD47 antibody

The anti-CD47 antibody of this example is the anti-CD47 monoclonal antibody disclosed in Patent Publication No. WO2019042285A1, which is incorporated herein by reference in its entirety for the purpose of the present application, wherein the anti-CD47 antibody is preferably the anti-CD47 antibody ADI-26630 disclosed in WO2019042285A1. The sequence of the anti-CD47 antibody in use is renumbered herein.

The amino acid sequences of the CDR regions, light and heavy chain variable regions, light and heavy chains of the exemplary anti-CD47 antibody of the present invention are listed in the SEQUENCE LISTING of the present application. In addition, the sequence numbers of the CDR regions and light and heavy chain variable regions of the above exemplary antibody of the present invention are shown in Table 2 and Table 3.

The anti-CD47 antibody of the present invention was expressed in and isolated from yeasts and CHO-S cells.

Expression in and isolation from yeast cells:

Yeast-based antibody presentation libraries (Adimab) were amplified according to prior art (described in WO2009036379; WO2010105256; WO2012009568), with a diversity of $1 \times 10^9$ in each library. Briefly, the first two rounds of screening employed magnetic-activated cell sorting using the MACS system available from Miltenyi. First, yeast cells (about $1 \times 10^{10}$ cells/library) from the libraries were separately incubated in FACS wash buffer (phosphate buffer, containing 0.1% bovine serum albumin) containing 100 nM biotin-labeled CD47 antigens (Acro Biosystems, Catalog No.: CD7-H5227-1mg) at room temperature for 15 min. The cells were washed once with 50 mL of pre-cooled FACS wash buffer, and resuspended with 40 mL of the same wash buffer, followed by addition of 500 μL of streptomycin microbeads (Miltenyi LS) and incubation at 4 °C for 15 min. The mixture was centrifuged at 1000 rpm for 5 min. After the supernatant was discarded, the cells were resuspended with 5 mL of FACS wash buffer. The resulting cell suspension was loaded on a Miltenyi LS column. After loading, the column was washed three times, with 3 mL of FACS wash buffer each time. The Miltenyi LS column was removed from the magnetic field and eluted with 5 mL of growth medium. The eluted yeast cells were collected and incubated overnight at 37 °C.

The next round of sorting was performed using a flow cytometer, wherein approximately $1 \times 10^8$ yeast cells screened by the MACS system were washed three times with FACS buffer and incubated in CD47 antigens labeled by a low concentration of biotin (100-1 nM) at room temperature. The culture solution was discarded. The cells were washed twice with FACS wash buffer, and mixed with LC-FITC (FITC-labeled goat anti-human immunoglobulin F(ab') kappa chain antibody, Southern Biotech) (1:100 dilution) and SA-633 (streptavidin-633, Molecular Probes) (1:500 dilution) or SA-PE (streptavidin-phycoerythrin, Sigma) (1:50 dilution) reagents, and the mixture was incubated at 4 °C for 15 min.

The cells were washed twice with pre-cooled FACS wash buffer, resuspended in 0.4 mL of buffer and transferred into a separator tube with a filter. The cells were sorted using FACS ARIA (BD Biosciences).

The yeast cells expressing the anti-CD47 antibody obtained by screening were induced by shaking at 30 °C for 48 h to express the anti-CD47 antibody. After the induction, the yeast cells were removed by centrifugation at 1300 rpm for 10 min, and the supernatant was collected. The anti-CD47 antibody in the supernatant was purified by Protein A affinity chromatography and eluted with an acetic acid solution pH 2.0, and the anti-CD47 antibody was harvested. The purity was greater than 95%. The antibody was digested using papain and purified by KappaSelect (GE Healthcare) to produce the corresponding Fab fragments.

Expression in and isolation from CHO-S cells:

According to the manufacturer's instructions, the Freedom® CHO-S® kit (Invitrogen) was used to produce the antibody-expressing CHO-S cell line. The DNA sequences of the heavy and light chains of the antibody molecule were first inserted into the same pCHO1.0 plasmid, with the heavy chain being upstream of the light chain. The constructed pCHO1.0 plasmids were then transferred into CHO cells by chemical transfection and electrotransfection, and the antibody yield was measured by ForteBio to determine the transfection efficiency 48 h after transfection. The transfected cells were subjected to two rounds of pressurized screening to give a cell pool of high antibody expression. The cell pool was then expanded to express a large quantity of antibodies, and the cell supernatant was collected and purified by Protein A affinity chromatography to a purity of > 95%.

2. Hu5F9

The control antibody Hu5F9 was expressed in and isolated from HEK293 cells: Hu5F9 is a human CD47 antibody transiently expressed in HEK293 cells, with its sequence being the same as that of antibody "5F9" in U.S. Patent No. US2015/0183874A1.

For transient expression of the antibody in HEK293 cells, the vector pTT5 (Addgene, Catalog No. 52326) was used. The heavy and light chain genes of the antibody were first cloned into separate pTT5 vectors. The pTT5 vectors carrying the heavy and light chain genes of the antibody molecule were transferred into the HEK293 cells by chemical transfection. The incubated HEK293 cells were transiently transfected using a chemical transfection reagent PEI (purchased from Polysciences) according to the method provided by the manufacturer. Plasmid DNAs and transfection reagent were prepared in a superclean bench, and then F17 medium (Gibco) (the volume was 1/5 of the transfection volume) was aliquoted into two 50-mL centrifuge tubes. The filtered plasmids (130 $\mu$g/100 mL) were added to one tube, and the filtered PEI (1 g/L, Polysciences) (mass ratio (plasmid:PEI) = 1:3) was added to the other. The two mixtures were each mixed well for 5 min, and then the two were mixed well and gently together for 20 times and let stand for 15-30 min (no more than 30 min). The DNA/PEI mixture was gently poured into the HEK293 cells and mixed well. The cells were incubated at 37 °C, 8% $CO_2$ for 7 days, with fresh culture medium being added every 48 h. Seven days later, or when the cells were continuously incubated to a cell viability $\leq$ 60%, the mixture was centrifuged at 13000 rpm for 20 min. The supernatant was purified by Protein A affinity chromatography to a purity of > 95%.

Heavy chain sequence of Hu5F9:

QVQLVQSGAEVKKPGASVKVSCKASGYTFTNYNMHWVRQAPGQRLEW

MGTIYPGNDDTSYNQKFKDRVTITADTSASTAYMELSSLRSEDTAVYYCA

RGGYRAMDYWGQGTLVTVSSASTKGPSVFPLAPCSRSTSESTAALGCLV

KDYFPEPVTVSWNSGALTSGVHTFPAVLQSSGLYSLSSVVTVPSSSLGTKT

YTCNVDHKPSNTKVDKRVESKYGPPCPPCPAPEFLGGPSVFLFPPKPKDTL

MISRTPEVTCVVVDVSQEDPEVQFNWYVDGVEVHNAKTKPREEQFNSTY

RVVSVLTVLHQDWLNGKEYKCKVSNKGLPSSIEKTISKAKGQPREPQVYT

LPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGK (SEQ ID NO: 13);

Light chain sequence of Hu5F9:

DIVMTQSPLSLPVTPGEPASISCRSSQSIVYSNGNTYLGWYLQKPGQSPQL LIYKVSNRFSGVPDRFSGSGSGTDFTLKISRVEAEDVGVYYCFQGSHVPY TFGQGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEV THQGLSSPVTKSFNRGEC (SEQ ID NO: 14).

3. Rituximab

Rituximab, an originator drug sold under the trade name of "MabThera", is produced jointly by Biogen and Roche. IBI301, a biosimilar of MabThera, is produced by Innovent Biologics (Suzhou) Co., Ltd., and was used as rituximab in following examples of the present invention.

The method for preparing the IBI301 antibody is as follows:

According to the manufacturer's instructions, the Freedom® CHO-S® kit (Invitrogen) was used to produce the antibody-expressing CHO-S cell line. The DNA sequences of the heavy and light chains of the antibody molecule were first inserted into the same pCHO1.0 plasmid, with the heavy chain being upstream of the light chain. The constructed pCHO1.0 plasmids were then transferred into CHO cells by chemical transfection and electrotransfection. The transfected cells were subjected to two rounds of pressurized screening to give a cell pool of high antibody expression. The cell pool was then expanded to express a large quantity of antibodies, and the cell supernatant was collected and purified by Protein A affinity chromatography to a purity of > 95%.

Heavy chain sequence of IBI301:

QVQLQQPGAELVKPGASVKMSCKASGYTFTSYNMHWVKQTPGRGLEWI GAIYPGNGDTSYNQKFKGKATLTADKSSSTAYMQLSSLTSEDSAVYYCAR STYYGGDWYFNVWGAGTTVTVSAASTKGPSVFPLAPSSKSTSGGTAALG

CLVKDYFPEPVTVSWNSGALTSGVHTFPAVLQSSGLYSLSSVVTVPSSSLG
TQTYICNVNHKPSNTKVDKKAEPKSCDKTHTCPPCPAPELLGGPSVFLFPP
KPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLS
LSPGK (SEQ ID NO: 15);

Light chain sequence of IBI301:

QIVLSQSPAILSASPGEKVTMTCRASSSVSYIHWFQQKPGSSPKPWIYATSN
LASGVPVRFSGSGSGTSYSLTISRVEAEDAATYYCQQWTSNPPTFGGGTK
LEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSS
PVTKSFNRGEC (SEQ ID NO: 16).

**Example 2. Establishment of Raji Tumor-Bearing Mouse Models**

The Raji lymphoma cells were dispersed with PBS (1 × ) to a cell density of $10 \times 10^6$/mL, and then mixed with Matrigel gel at a volume ratio of 1:1 to obtain a cell suspension with a cell density of $5 \times 10^6$/mL.

The right back of NOD-SCID mice was shaved and grafted by subcutaneous injection with the above cell suspension at a dose of $1 \times 10^6$ cells/0.2 mL/mouse.

On day 7 after grafting, 77 mice that were successfully modeled (with a tumor volume between 44.30 mm$^3$ and 97.65 mm$^3$) were picked and grouped in an S-shaped manner into 11 groups of 7, and the dosage regimen for each group is as follows: PBS group: PBS was intraperitoneally injected into the mice at a volume of 10 mL/kg once every two days for 6 doses.

h-IgG group: h-IgG was dissolved in PBS to 1.62 mg/mL and intraperitoneally injected into the mice at a volume of 10 mL/kg once every two days for 6 doses. Hu5F9-0.02 mg/kg group: Hu5F9 was dissolved in PBS to 0.002 mg/mL and intraperitoneally injected into the mice at a volume of 10 mL/kg once every two days for 6 doses.

Hu5F9-0.1 mg/kg group: Hu5F9 was dissolved in PBS to 0.01 mg/mL and intraperitoneally injected into the mice at a volume of 10 mL/kg once every two days for 6 doses.

Anti-CD47 antibody-0.02 mg/kg group: The anti-CD47 antibody was dissolved in PBS to 0.002 mg/mL and intraperitoneally injected into the mice at a volume of 10 mL/kg once every two days for 6 doses.

Anti-CD47 antibody-0.1 mg/kg group: The anti-CD47 antibody was dissolved in PBS to 0.01 mg/mL and intraperitoneally injected into the mice at a volume of 10 mL/kg once every two days for 6 doses.

Rituximab-1.5 mg/kg group: Rituximab was dissolved in PBS to 0.15 mg/mL and intraperitoneally injected into the mice at a volume of 10 mL/kg once every two days for 6 doses.

Hu5F9 + rituximab-0.02 mg/kg + 1.5 mg/kg group: Hu5F9 and rituximab were dissolved in PBS to prepare a solution containing Hu5F9 at 0.002 mg/mL and rituximab at 0.15 mg/mL, which was then intraperitoneally injected into the mice at a volume of 10 mL/kg once every two days for 6 doses.

Hu5F9 + rituximab-0.1 mg/kg + 1.5 mg/kg group: Hu5F9 and rituximab were dissolved in PBS to prepare a solution containing Hu5F9 at 0.01 mg/mL and rituximab at 0.15 mg/mL, which was then intraperitoneally injected into the mice at a volume of 10 mL/kg once every two days for 6 doses.

Anti-CD47 antibody + rituximab-0.02 mg/kg + 1.5 mg/kg group: The anti-CD47 antibody and rituximab were dissolved in PBS to prepare a solution containing the anti-CD47 antibody at 0.002 mg/mL and rituximab at 0.15 mg/mL, which was then intraperitoneally injected into the mice at a volume of 10 mL/kg once every two days for 6 doses.

Anti-CD47 antibody + rituximab-0.1 mg/kg + 1.5 mg/kg group: The anti-CD47 antibody and rituximab were dissolved in PBS to prepare a solution containing the anti-CD47 antibody at 0.01 mg/mL and rituximab at 0.15 mg/mL, which was then intraperitoneally injected into the mice at a volume of 10 mL/kg once every two days for 6 doses.

The mice was monitored for body weight and the maximum length of major axis (L) and maximum length of minor axis (Ws) of the tumor tissue twice a week for 4 weeks. After the end of experiment, the relative tumor growth inhibition for mice in various groups was calculated.

Measurement of tumor volume:

The maximum length of major axis (L) and maximum length of minor axis (W) of tumors were measured with a vernier caliper, and tumor volume was calculated using the following formula: $V = L \times W^2/2$.

Calculation of the relative tumor growth inhibition:

$$\text{Tumor growth inhibition TGI (\%)} = 100\% \times (\text{final tumor volume of the control group after treatment - final tumor volume of the treatment group after treatment})/(\text{final tumor volume of the control group after treatment - baseline tumor volume of the control group before treatment})$$

wherein the control group is the h-IgG group.

The tumor growth inhibitions of various groups are shown in Table 4.

Table 4. Tumor growth inhibition (TGI)%

| Groups | Tumor volume on day 28 after grafting (mm$^3$) (mean $\pm$ standard deviation) | TGI % | Number of complete tumor regressions (number of tumor regressions/total number of tumors) |
|---|---|---|---|
| PBS | 1000.78$\pm$142.64 | N/A | 0/7 |
| h-IgG | 826.87$\pm$506.98 | N/A | 0/7 |
| Hu5F9-0.02 mg/kg | 926.52$\pm$575.87 | -13% | 0/7 |
| Hu5F9-0.1 mg/kg | 672.38$\pm$197.18 | 20% | 0/7 |
| Anti-CD47 antibody-0.02 mg/kg | 644.81$\pm$253.81 | 24% | 0/7 |
| Anti-CD47 antibody-0.1 mg/kg | 436.45$\pm$384.68 | 52% | 1/7 |
| Rituximab-1.5 mg/kg | 826.99$\pm$478.9 | 0% | 0/7 |
| Hu5F9 + rituximab-0.02 mg/kg + 1.5 mg/kg | 576.81$\pm$507.72 | 33% | 1/7 |
| Hu5F9 + rituximab-0.1 mg/kg + 1.5 mg/kg | 618.32$\pm$352.98 | 28% | 0/7 |
| Anti-CD47 antibody + rituximab-0.02 mg/kg + 1.5 mg/kg | 405.82$\pm$184.83 | 56% | 0/7 |
| Anti-CD47 antibody + rituximab-0.1 mg/kg + 1.5 mg/kg | 127.85$\pm$176.61 | 92% | 4/7 |

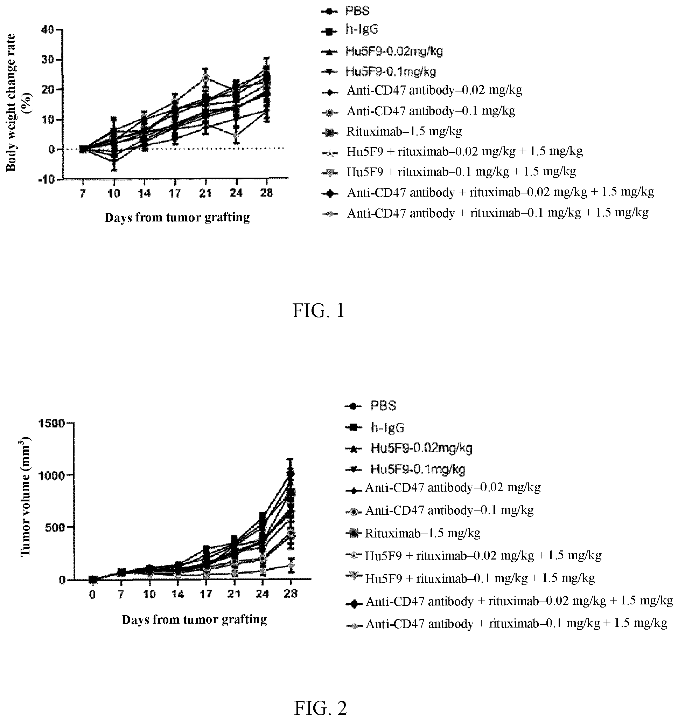

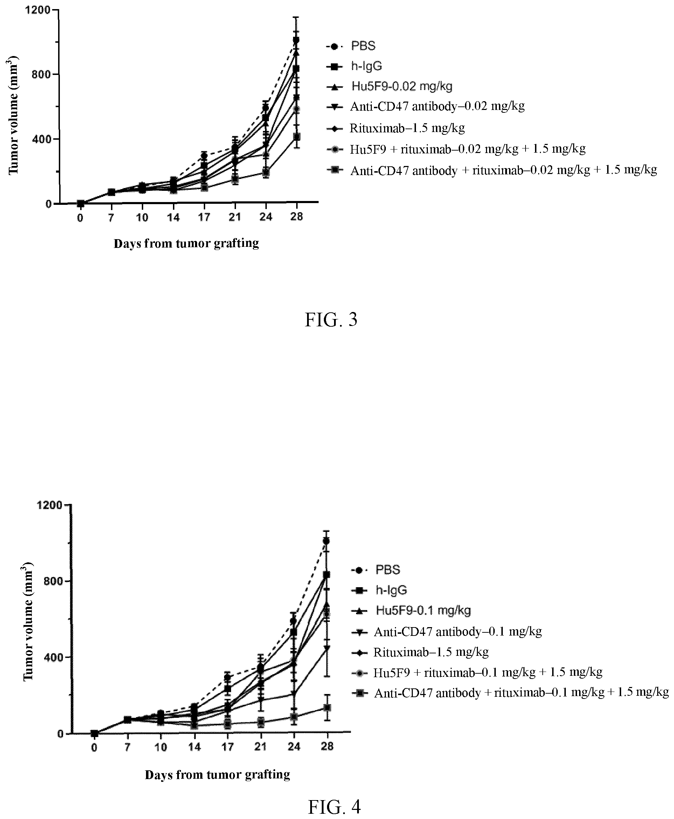

The body weight change rates of tumor-bearing mice in various groups are shown in FIG. 1. The tumor volume inhibitions of tumor-bearing mice in various groups are shown in FIG. 2. The tumor volume inhibitions of tumor-bearing mice in low dose groups and high dose groups are shown in FIG. 3 and FIG. 4, respectively.

It can be seen from Table 4 that, after the 6 doses, compared with the PBS group and the h-IgG group, the anti-CD47 antibody-0.1 mg/kg group, the anti-CD47 antibody + rituximab-0.02 mg/kg + 1.5 mg/kg group and the anti-CD47 antibody + rituximab-0.1 mg/kg + 1.5 mg/kg group demonstrated significant inhibitory effect on tumor growth of Raji tumor-bearing mice, resulting in smaller tumor sizes; the efficacy of the anti-CD47 antibody + rituximab-0.1 mg/kg + 1.5 mg/kg group was better than that of the anti-CD47 antibody + rituximab-0.02 mg/kg + 1.5 mg/kg group, indicating a dose-dependent anti-tumor efficacy for the anti-CD47 antibody. In addition, the tumor growth inhibitions of the anti-CD47 antibody + rituximab-0.02 mg/kg + 1.5 mg/kg group and the anti-CD47 antibody + rituximab-0.1 mg/kg + 1.5 mg/kg group were much higher than those of the Hu5F9 + rituximab-0.02 mg/kg + 1.5 mg/kg group and the Hu5F9 + rituximab-0.1 mg/kg + 1.5 mg/kg group.

The Hu5F9-0.02 mg/kg group and rituximab-1.5 mg/kg group showed no anti-tumor efficacy compared with the h-IgG group; the tumor growth inhibitions of the Hu5F9-0.1 mg/kg group, the anti-CD47 antibody-0.02 mg/kg group and the anti-CD47 antibody-0.1 mg/kg group were 20%, 24% and 52%, respectively; the tumor growth inhibitions of the Hu5F9 + rituximab-0.02 mg/kg + 1.5 mg/kg group, the Hu5F9 + rituximab-0.1 mg/kg + 1.5 mg/kg group, the anti-CD47 antibody + rituximab-0.02 mg/kg + 1.5 mg/kg group and the anti-CD47 antibody + rituximab-0.1 mg/kg + 1.5 mg/kg group were 33%, 28%, 56% and 92%, respectively.

The tumor inhibition effect in mice of the anti-CD47 antibody + rituximab-0.02 mg/kg + 1.5 mg/kg group was stronger than those in mice of the anti-CD47 antibody-0.02 mg/kg group, the rituximab-1.5 mg/kg group and the Hu5F9 + rituximab-0.02 mg/kg + 1.5 mg/kg group; the tumor inhibition effect in mice of the anti-CD47 antibody + rituximab-0.1 mg/kg + 1.5 mg/kg group was stronger than those in mice of the anti-CD47 antibody-0.1 mg/kg group, the rituximab-1.5 mg/kg group and the Hu5F9 + rituximab-0.1 mg/kg + 1.5 mg/kg group. In conclusion, the above results suggested that the anti-CD47 antibody + rituximab combinations have superior tumor inhibition effect to the anti-CD47 antibody monotherapies and the rituximab monotherapies and demonstrate a combination effect, and the tumor inhibition effect in the anti-CD47 antibody + rituximab combination groups is better than that of the Hu5F9 + rituximab combination groups at the same dose. In the anti-CD47 antibody-0.1 mg/kg group, 1 mouse showed complete tumor regression; in the Hu5F9 + rituximab-0.02 mg/kg + 1.5 mg/kg group, 1 mouse showed complete tumor regression; in the anti-CD47 antibody + rituximab-0.1 mg/kg + 1.5 mg/kg group, 4 mice showed complete tumor regression, indicating extremely strong tumor inhibition effect.

It can be seen from FIG. 1 that, no significant toxic and side effect was found for the monotherapies or combination of the anti-CD47 antibody and rituximab in the preclinical mouse model, and the mice demonstrated normal body weights without decrease.

It can be seen from FIG. 2, 3 and 4 and Table 4 that, the anti-CD47 antibody has certain anti-tumor efficacy which was significantly enhanced by combined use with rituximab, indicating that the two drugs have synergistic effect in combination. In addition, at the same dose, the combination of the anti-CD47 antibody and rituximab has better tumor inhibition effect in the tumor-bearing mice than the combination of Hu5F9 and rituximab.

## Claims

1. Use of a combination of an anti-CD47 antibody or an antigen-binding fragment thereof and an anti-CD20 antibody in preparing a medicament for preventing and/or treating a tumor and/or cancer, wherein the anti-CD47 antibody is a fully human anti-CD47 monoclonal antibody;
the fully human anti-CD47 monoclonal antibody comprises a heavy chain variable region VH and a light chain variable region VL, wherein the VH comprises complementarity determining regions HCDR1, HCDR2 and HCDR3, the HCDR1 having an amino acid sequence set forth in SEQ ID NO: 3 or SEQ ID NO: 11, the HCDR2 having an amino acid sequence set forth in SEQ ID NO: 4, the HCDR3 having an amino acid sequence set forth in SEQ ID NO: 5 or SEQ ID NO: 12; the VL comprises complementarity determining regions LCDR1, LCDR2 and LCDR3, the LCDR1 having an amino acid sequence set forth in SEQ ID NO: 6, the LCDR2 having an amino acid sequence set forth in SEQ ID NO: 7, the LCDR3 having an amino acid sequence set forth in SEQ ID NO: 8.

2. The use according to claim 1, wherein the heavy chain variable region VH comprises an amino acid sequence set forth in SEQ ID NO: 9 or a sequence having at least 90%, 95%, 98% or 99% identity thereto, and the light chain variable region VL comprises a sequence set forth in SEQ ID NO: 10 or a sequence having at least 90%, 95%, 98% or 99% identity thereto;
preferably, the heavy chain variable region VH has an amino acid sequence set forth in SEQ ID NO: 9, and the light chain variable region VL has an amino acid sequence set forth in SEQ ID NO: 10.

3. The use according to claim 1, wherein the fully human anti-CD47 monoclonal antibody comprises a heavy chain comprising an amino acid sequence set forth in SEQ ID NO: 1 or an amino acid sequence having at least 90%, 95%, 98% or 99% identity thereto, and a light chain comprising an amino acid sequence set forth in SEQ ID NO: 2 or an amino acid sequence having at least 90%, 95%, 98% or 99% identity thereto;
preferably, the heavy chain has an amino acid sequence set forth in SEQ ID NO: 1, and the light chain has an amino acid sequence set forth in SEQ ID NO: 2.

4. The use according to any one of claims 1 to 3, wherein the anti-CD20 antibody is an anti-CD20 monoclonal antibody;

   preferably, the anti-CD20 monoclonal antibody is obinutuzumab, ibritumomab tiuxetan, ofatumumab, tositumomab, ocrelizumab or rituximab;
   preferably, the anti-CD20 monoclonal antibody is rituximab.

5. A single pharmaceutical dosage unit, comprising effective amounts of the anti-CD47 antibody or the antigen-binding fragment thereof and the anti-CD20 antibody according to any one of claims 1 to 4.

6. A pharmaceutical composition, comprising an effective amount of the combination of the anti-CD47 antibody or the antigen-binding fragment thereof and the anti-CD20 antibody according to any one of claims 1 to 4, and a pharmaceutically acceptable carrier.

7. A kit of parts, comprising an effective amount of the combination of the anti-CD47 antibody or the antigen-binding fragment thereof and the anti-CD20 antibody according to any one of claims 1 to 4, the single pharmaceutical dosage unit according to claim 5, or the pharmaceutical composition according to claim 6, wherein:
   preferably, in the same package, the kit comprises:

   - a first container containing a pharmaceutical composition for parenteral administration comprising the anti-CD47 antibody; and
   - a second container containing a pharmaceutical composition for parenteral administration comprising the anti-CD20 antibody.

8. Use of the combination of the anti-CD47 antibody or the antigen-binding fragment thereof and the anti-CD20 antibody according to any one of claims 1 to 4, the single pharmaceutical dosage unit according to claim 5, the pharmaceutical composition according to claim 6, or the kit of parts according to claim 7, in preparing a medicament for preventing and/or treating a tumor and/or cancer, wherein:
   preferably, the tumor and/or cancer is a hematological tumor, more preferably lymphoma, and even more preferably CD20-positive non-Hodgkin's lymphoma.

9. Use of an anti-CD47 antibody in preparing a product for enhancing an efficacy of an anti-CD20 antibody in preventing and/or treating a tumor and/or cancer, wherein:

   preferably, the anti-CD47 antibody is the anti-CD47 antibody according to any one of claims 1 to 3;
   preferably, the anti-CD20 antibody is the anti-CD20 antibody according to claim 4;
   preferably, the tumor is a hematological tumor, more preferably lymphoma, and even more preferably CD20-positive non-Hodgkin's lymphoma.

10. A method for preventing and/or treating a tumor and/or cancer, comprising: administering to an individual an effective amount of the combination of the anti-CD47 antibody or the antigen-binding fragment thereof and the anti-CD20 antibody according to any one of claims 1 to 4, the single pharmaceutical dosage unit according to claim 5, the pharmaceutical composition according to claim 6 or the kit of parts according to claim 7.

FIG. 1

FIG. 2

FIG. 3

FIG. 4

## INTERNATIONAL SEARCH REPORT

| International application No. |
|---|
| **PCT/CN2021/070493** |

**A. CLASSIFICATION OF SUBJECT MATTER**

C07K 16/28(2006.01)i; A61K 39/395(2006.01)i; A61P 35/00(2006.01)i; A61K 39/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS: CD47, CD20, 整联蛋白相关蛋白, IAP, 抗体, 联合, 组合, 肿瘤, 癌症, 利妥昔单抗, ADI-26630, rituximab, CDR, antibody, combination, SEQ ID NOs:1-10, 信达生物制药(苏州)有限公司, 陈炳良, 吴敏, 伍伟伟

**C. DOCUMENTS CONSIDERED TO BE RELEVANT**

| Category* | Citation of document, with indication, where appropriate, of the relevant passages | Relevant to claim No. |
|---|---|---|
| Y | INNOVENT BIOLOGICS SUZHOU CO., LTD. "NCT03717103" *ClinicalTrials.gov*, 19 December 2018 (2018-12-19), "Study Description", and "Arms and Interventions" | 1-10 |
| Y | "RN number:2283356-07-8" *STN Registry*, 11 March 2019 (2019-03-11), sequence | 1-10 |
| Y | CN 109422811 A (INNOVENT BIOLOGICS (SUZHOU) CO., LTD.) 05 March 2019 (2019-03-05) description, tables 1 and 2, and embodiments 4-6 | 1-10 |
| Y | CN 110612309 A (CELGENE CORPORATION) 24 December 2019 (2019-12-24) description, embodiments 1 and 2 | 1-10 |
| Y | CN 110526972 A (JIANGSU HENGRUI MEDICINE CO., LTD. et al.) 03 December 2019 (2019-12-03) description, specific embodiment | 1-10 |
| Y | CN 108290948 A (ERASMUS UNIVERSITY MEDICAL CENTER et al.) 17 July 2018 (2018-07-17) description, embodiment 3 | 1-10 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 March 2021** | **06 April 2021** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/070493**

### C.    DOCUMENTS CONSIDERED TO BE RELEVANT

| Category* | Citation of document, with indication, where appropriate, of the relevant passages | Relevant to claim No. |
|---|---|---|
| A | WO 2019157432 A1 (FORTY SEVEN, INC.) 15 August 2019 (2019-08-15)<br>claims 1-14 | 1-10 |
| A | WO 2014087248 A2 (NOVIMMUNE S.A.) 12 June 2014 (2014-06-12)<br>entire document | 1-10 |
| A | CN 110087673 A (TEVA PHARMACEUTICALS AUSTRALIA PTY LTD.) 02 August 2019<br>(2019-08-02)<br>entire document | 1-10 |

Form PCT/ISA/210 (second sheet) (January 2015)

**INTERNATIONAL SEARCH REPORT**

International application No.

**PCT/CN2021/070493**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

<div style="text-align:center">**INTERNATIONAL SEARCH REPORT**</div>

| International application No. |
| --- |
| **PCT/CN2021/070493** |

**Box No. II      Observations where certain claims were found unsearchable (Continuation of item 2 of first sheet)**

This international search report has not been established in respect of certain claims under Article 17(2)(a) for the following reasons:

1.  ☑ Claims Nos.: **10**
     because they relate to subject matter not required to be searched by this Authority, namely:

     [1]   Claim 10 relates to a method for the prevention and/or treatment of tumors and/or cancer, i.e. claim
           10 relates to the subject matter defined in PCT Rule 39.1 that does not warrant an international search
           unit search: (4) methods for treatment of the human or animal body by surgery or therapy, as well as
           diagnostic methods performed on a human or animal body. The international search is made on the
           basis of the use of an effective amount of said combination of anti-CD47 antibody or antigen-binding
           fragment thereof and anti-CD20 antibody, said single drug dose unit, said drug composition or said kit
           in the preparation of a drug for the treatment of cancer.

2.  ☐ Claims Nos.:
     because they relate to parts of the international application that do not comply with the prescribed requirements to such an
     extent that no meaningful international search can be carried out, specifically:

3.  ☐ Claims Nos.:
     because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/070493**

| Patent document cited in search report | | | Publication date (day/month/year) | Patent family member(s) | | | Publication date (day/month/year) |
|---|---|---|---|---|---|---|---|
| CN | 109422811 | A | 05 March 2019 | AU | 2018325845 | B2 | 26 November 2020 |
| | | | | EP | 3546482 | A4 | 21 October 2020 |
| | | | | KR | 20190105024 | A | 11 September 2019 |
| | | | | JP | 2020508645 | A | 26 March 2020 |
| | | | | WO | 2019042285 | A1 | 07 March 2019 |
| | | | | EP | 3546482 | A1 | 02 October 2019 |
| | | | | CA | 3048578 | A1 | 07 March 2019 |
| | | | | AU | 2018325845 | A1 | 11 July 2019 |
| | | | | US | 2020181259 | A1 | 11 June 2020 |
| | | | | BR | 112019014694 | A2 | 07 April 2020 |
| | | | | CN | 110214154 | A | 06 September 2019 |
| CN | 110612309 | A | 24 December 2019 | CA | 3057841 | A1 | 04 October 2018 |
| | | | | WO | 2018183182 | A1 | 04 October 2018 |
| | | | | KR | 20190133198 | A | 02 December 2019 |
| | | | | JP | 2020515577 | A | 28 May 2020 |
| | | | | IL | 269590 | D0 | 28 November 2019 |
| | | | | US | 2020330590 | A1 | 22 October 2020 |
| | | | | CO | 2019011640 | A2 | 18 February 2020 |
| | | | | CL | 2019002716 | A1 | 29 May 2020 |
| | | | | MX | 2019011624 | A | 05 December 2019 |
| | | | | EP | 3601351 | A1 | 05 February 2020 |
| | | | | AU | 2018244276 | A1 | 17 October 2019 |
| | | | | WO | 2018183182 | A8 | 14 November 2019 |
| | | | | EA | 201992278 | A1 | 03 March 2020 |
| | | | | SG | 11201908678X | A | 30 October 2019 |
| | | | | BR | 112019020185 | A2 | 02 June 2020 |
| CN | 110526972 | A | 03 December 2019 | None | | | |
| CN | 108290948 | A | 17 July 2018 | US | 2017081407 | A1 | 23 March 2017 |
| | | | | CA | 2999277 | A1 | 30 March 2017 |
| | | | | US | 9650441 | B2 | 16 May 2017 |
| | | | | US | 2018201677 | A1 | 19 July 2018 |
| | | | | US | 10570201 | B2 | 25 February 2020 |
| | | | | US | 9803016 | B2 | 31 October 2017 |
| | | | | GB | 2558131 | A | 04 July 2018 |
| | | | | IL | 258257 | D0 | 31 May 2018 |
| | | | | GB | 201806466 | D0 | 06 June 2018 |
| | | | | US | 2017204181 | A1 | 20 July 2017 |
| | | | | US | 10442858 | B2 | 15 October 2019 |
| | | | | US | 2018105591 | A1 | 19 April 2018 |
| | | | | JP | 2018535692 | A | 06 December 2018 |
| | | | | WO | 2017053423 | A1 | 30 March 2017 |
| | | | | HK | 1257310 | A1 | 18 October 2019 |
| | | | | US | 2020291114 | A1 | 17 September 2020 |
| | | | | AU | 2016326423 | A1 | 26 April 2018 |
| | | | | HK | 1257309 | A1 | 18 October 2019 |
| | | | | EP | 3353209 | A1 | 01 August 2018 |
| WO | 2019157432 | A1 | 15 August 2019 | KR | 20200119834 | A | 20 October 2020 |
| | | | | US | 2019248915 | A1 | 15 August 2019 |
| | | | | CA | 3090489 | A1 | 15 August 2019 |
| | | | | AU | 2019218271 | A1 | 10 September 2020 |

Form PCT/ISA/210 (patent family annex) (January 2015)

**INTERNATIONAL SEARCH REPORT**
**Information on patent family members**

International application No.

**PCT/CN2021/070493**

| Patent document cited in search report | | | Publication date (day/month/year) | Patent family member(s) | | | Publication date (day/month/year) |
|---|---|---|---|---|---|---|---|
| | | | | EP | 3752190 | A1 | 23 December 2020 |
| | | | | CN | 111699005 | A | 22 September 2020 |
| | | | | WO | 2019157432 | A8 | 25 June 2020 |
| WO | 2014087248 | A2 | 12 June 2014 | RU | 2015126533 | A | 12 January 2017 |
| | | | | RU | 2019118257 | A | 24 June 2019 |
| | | | | DK | 2925782 | T3 | 06 April 2020 |
| | | | | EP | 3725807 | A1 | 21 October 2020 |
| | | | | JP | 2019023187 | A | 14 February 2019 |
| | | | | US | 2018291115 | A1 | 11 October 2018 |
| | | | | US | 2018291116 | A1 | 11 October 2018 |
| | | | | JP | 6392770 | B2 | 19 September 2018 |
| | | | | ES | 2784631 | T3 | 29 September 2020 |
| | | | | PT | 2925782 | T | 22 April 2020 |
| | | | | EP | 2925782 | A2 | 07 October 2015 |
| | | | | US | 2018355065 | A1 | 13 December 2018 |
| | | | | RU | 2693078 | C2 | 01 July 2019 |
| | | | | CN | 105121467 | B | 05 July 2019 |
| | | | | JP | 2020183451 | A | 12 November 2020 |
| | | | | CN | 105121467 | A | 02 December 2015 |
| | | | | AU | 2013353763 | B2 | 13 September 2018 |
| | | | | US | 2014303354 | A1 | 09 October 2014 |
| | | | | AU | 2018220150 | A1 | 13 September 2018 |
| | | | | JP | 2015536351 | A | 21 December 2015 |
| | | | | WO | 2014087248 | A3 | 06 November 2014 |
| | | | | CN | 110183534 | A | 30 August 2019 |
| | | | | AU | 2013353763 | A1 | 11 June 2015 |
| | | | | EP | 2925782 | B1 | 22 January 2020 |
| | | | | CA | 2892585 | A1 | 12 June 2014 |
| CN | 110087673 | A | 02 August 2019 | US | 2020010544 | A1 | 09 January 2020 |
| | | | | WO | 2018014067 | A9 | 24 January 2019 |
| | | | | JP | 2019521169 | A | 25 July 2019 |
| | | | | EP | 3487522 | A1 | 29 May 2019 |
| | | | | WO | 2018014067 | A1 | 25 January 2018 |
| | | | | EP | 3487522 | A4 | 01 April 2020 |
| | | | | CA | 3030926 | A1 | 25 January 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019042285 A1
- WO 2009036379 A
- WO 2010105256 A
- WO 2012009568 A
- US 20150183874 A1

### Non-patent literature cited in the description

- Fundamental Immunology. Raven Press, 1993
- Antibody Drug Research and Application. People's Medical Publishing House, 2013
- **HOLLINGER et al.** *PNAS USA,* 1993, vol. 90, 6444-6448
- **HUDSON et al.** *Nat. Med.,* 2003, vol. 9, 129-134
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. U.S. Department of Health and Human Services, National Institutes of Health, 1987
- **KABAT et al.** Sequences of Proteins of Immunological Interest. Public Health Service, National Institutes of Health, 1991
- **NEEDLEMA ; WUNSCH.** *J. Mol. Biol.,* 1970, vol. 48, 444-453, http://www.gcg.com
- **E. MEYERS ; W. MILLER.** *CABIOS,* 1989, vol. 4, 11-17